# EUROPÄISCHE PATENTANMELDUNG

(11) **EP 3 139 157 A1**
(43) Veröffentlichungstag der Anmeldung: **08.03.2017**
(21) Anmeldenummer: 16182302.6
(22) Anmeldetag: 02.08.2016
(51) Int. Cl.: G01N 23/18, G01N 33/44

(54) **VORRICHTUNG UND VERFAHREN ZUR RADIOSKOPISCHEN UNTERSUCHUNG EINES STREIFENFÖRMIGEN MATERIALS MIT EINEM WESENTLICHEN BESTANDTEIL AUS KAUTSCHUK ODER KUNSTSTOFF**

(30) Priorität: 02.09.2015 DE 102015114658
(71) Anmelder: TROESTER GmbH & Co. KG, 30519 Hannover (DE)
(72) Erfinder: Pielsticker, Bernd, 31515 Wunstorf (DE); Schoppmann, Kurt, 30519 Hannover (DE)
(74) Vertreter: Scheffler, Jörg

(57) **Zusammenfassung**

Die Erfindung betrifft eine Vorrichtung (1) und ein Verfahren zur radioskopischen Untersuchung eines insbesondere kontinuierlich durchlaufenden, endlosen streifenförmigen Materials (3) aus Kautschuk. Während der Bewegung wird das streifenförmige Material (3) von einer radioskopischen Messeinrichtung (2) durchleuchtet und dabei die gesamte Querschnittsfläche erfasst, sodass in dem Material (3) vorhandene Fremdkörper oder Fehlstellen nach ihrer Position und Orientierung erfasst werden. Mit einer Ausscheidevorrichtung (8) wird der zuvor identifizierte Fremdkörper während der Vorschubbewegung des Materials (3) entfernt, indem ein als Stanzwerkzeug ausgeführtes Werkzeug (10) der Ausscheidevorrichtung (8) synchron zu dem Material (3) bewegt wird.

## Beschreibung

Die Erfindung betrifft eine Vorrichtung mit einer radioskopischen Messeinrichtung zur Untersuchung eines insbesondere kontinuierlich durchlaufenden, endlosen streifenförmigen Materials mit einem wesentlichen Bestandteil aus Kautschuk oder Kunststoff, wobei das Material mittels eines eine umlaufend antreibbare Auflage aufweisenden Fördermittels zuführbar ist. Weiterhin betrifft die Erfindung ein Verfahren zur radioskopischen Untersuchung des Materials.

In der modernen Reifenproduktion wird bei der zerstörungsfreien Materialprüfung die digitale Radioskopie bereits vielfach eingesetzt und zählt somit bereits aufgrund offenkundiger Vorbenutzung zum Stand der Technik.

Dabei erfolgt die Materialprüfung in Form von Stichproben-Kontrollen oder aber die Materialprüfung wird direkt eingebunden in den Produktionsprozess. Besonders hohen Anforderungen unterliegt dabei die Reifenfertigung, die aus mehr als 200 verschiedenen Materialien zusammengesetzt sein kann und hohen Qualitätsansprüchen unterliegt.

Die typische Prüfaufgabe für die Röntgenprüfung umfasst in der Hauptsache den Nachweis der Symmetrie des inneren Reifenaufbaues. Dabei wird der Lagenversatz einzelner Gürtel, die Stahlkordlagen sowie die Kordabstände in der Karkasse überprüft. Weiterhin werden die Reifen auf Luft- und Fremdkörper oder Fehlstellen-Einschlüsse untersucht. Dabei kann ein Röntgenbild des kompletten Reifens durch Rotation erzeugt werden.

Zur Qualifizierung der Bildgüte für radioskopische Systeme für die Reifenprüfung wird beispielsweise der ASTM Standard F-1035 eingesetzt. Dabei handelt es sich um eine flache Scheibe aus einer definierten Gummi-Mischung, in der mehrere Materialien in bestimmten Strukturen eingebracht sind und die jeweils im Röntgenbild differenziert werden können.

Die DE 196 07 582 A1 offenbart ein Verfahren zur Überwachung eines extrudierten Bandes. Hierbei werden kleine Bereiche des Bandes kontinuierlich oder schrittweise quer zur Bandebene mit Röntgenstrahlung durchstrahlt und die Art, die Größe, die Form und/oder die Verteilung der Verunreinigungen sowohl im Bandinneren als auch auf der Bandoberfläche erfasst. Überschreiten die Verunreinigungen einen vorgegebenen Schwellwert, so wird das disperse System von seiner Weiterverarbeitung ausgeschlossen.

Aus der DE 971 619 B ist eine Einrichtung zur Kontrolle der Wandungen bei der Fertigung von Schläuchen und Rohren aus Kunststoff mittels radioaktiver Strahler bekannt.

Die DE 101 60 398 B4 bezieht sich auf ein Verfahren zur Prüfung einer mittels eines Fördermittels bewegten Matte aus Biomassepartikeln, insbesondere aus Fasern oder Holzspänen, zur Herstellung von Spanplatten. Dabei werden die Biomassepartikel mittels einer Streumaschine auf ein Formband gestreut. Die vorkomprimierte Matte durchläuft anschließend eine mit Strahlungsquellen ausgestattete Prüfvorrichtung zur Durchstrahlung der Matte, die so auf Fremdkörper, wie Metall- oder Kunststoffteile untersucht wird. Das Formband ist geteilt und lässt sich, wenn durch die Prüfvorrichtung ein Fehler in der Matte festgestellt worden ist, unter Bildung eines Spaltes auseinanderfahren, sodass der den Fehler enthaltende Abschnitt der Matte in einen Abwurfschacht entsorgt wird.

Die DE 10 2009 012 558 A1 betrifft eine Kontrollvorrichtung einer Produktionsmaschine einer Abfüllanlage. Um das Vorhandensein von Fremdkörpern in den abgefüllten Bechern auszuschließen, werden die vereinzelten und verpackten Becher mittels einer Röntgenkontrollvorrichtung untersucht.

In der Praxis wird aufgrund eines erfassten Fremdkörpers in dem Material die Materialzufuhr unterbrochen, sodass ein den Fremdkörper oder Fehlstellen einschließender Materialabschnitt manuell entfernt werden kann. Die Materialzuführung und damit die sich anschließenden Verarbeitungsprozesse des Materials werden dadurch unterbrochen und erfordern zusätzliche Anlaufmaßnahmen zur Fortsetzung des Prozesses.

Ausgehend von diesem Stand der Technik liegt der Erfindung die Aufgabe zugrunde, eine Möglichkeit zu schaffen, den Produktionsprozess insbesondere bei der Verarbeitung strang- oder streifenförmiger Materialien weiter zu verbessern.

Diese Aufgabe wird erfindungsgemäß mit einer Vorrichtung gemäß den Merkmalen des Anspruches 1 gelöst. Die weitere Ausgestaltung der Erfindung ist den Unteransprüchen zu entnehmen.

Erfindungsgemäß ist also bei der Vorrichtung die Messeinrichtung zur Erfassung bzw. Durchleuchtung der gesamten Querschnittsfläche ausgeführt, sodass in dem Material vorhandene Fremdkörper oder Fehlstellen einschließlich möglicher Verunreinigungen sowie von Sollwerten abweichenden Materialeigenschaften oder -parameter nach ihrer Größe, Position und/oder Orientierung in dem Material erfassbar sind und dass die Vorrichtung mit einer Ausscheidevorrichtung zur Entfernung des Fremdkörpers oder der Fehlstelle oder alternativ oder zusätzlich mit einer Markiervorrichtung zur Markierung des Fremdkörpers oder der Fehlstelle aufgrund der Positionsdaten zusammen mit einem den Fremdkörper oder die Fehlstelle einschließenden Teilbereich der Querschnittsfläche ausgestattet ist. Hierdurch wird in besonders vorteilhafter Weise, insbesondere während das Material durch das eine umlaufend antreibbare Auflage aufweisende Fördermittel kontinuierlich bewegt wird, ein erkannter Fremdkörper oder eine Fehlstelle in dem Material nicht nur erkannt, sondern unmittelbar entfernt. Hierzu wird nicht nur die Position in Längs- und Querrichtung, also der Abstand von den Seitenrändern bestimmt. Vielmehr wird dabei auch die Tiefenkoordinate des Fremdkörpers oder der Fehlstelle in der Querschnittsebene ermittelt, sodass der zu entfernende Teilbereich örtlich optimal eingegrenzt werden kann. Ausgehend von einer Oberseite oder Unterseite, abhängig von dem erfassten Abstand von der Oberfläche des streifenförmigen Materials, wird der Teilbereich vorzugsweise nicht als Durchbrechung des Materials, sondern beschränkt auf notwendige Ebenen abgehoben und entfernt. Hierzu eignen sich beispielsweise Zangen- oder scherenartige Werkzeuge. Somit wird der damit verbundene Materialverlust auf ein Minimum reduziert, indem das Materialvolumen des Teilbereiches nicht durch die gesamte Materialstärke umfasst, sondern auf eine vorzugsweise oberflächennahe Schicht beschränkt ist.

Dabei hat es sich bereits als besonders praxisgerecht erwiesen, wenn die Ausscheidevorrichtung zur Entfernung des Teilbereiches während einer Vorschubbewegung des Materials erfolgt. Hierzu wird zumindest ein Trennwerkzeug der Ausscheidevorrichtung gemeinsam mit dem Material, insbesondere in Verbindung mit dem Fördermittel während des Trennvorganges des Teilbereiches mit dem Material mitbewegt. Hierdurch können unerwünschte Spannungen und Verformungen in dem Material vermieden werden.

Eine besonders vorteilhafte Ausgestaltung der vorliegenden Erfindung wird auch dadurch erreicht, dass die Ausscheidevorrichtung zumindest ein Stanzwerkzeug zum Ausstanzen des Teilbereiches aufweist. Insbesondere eignet sich hierbei ein hohlzylindrisches Stanzwerkzeug, welches mit einem von dem Stanzwerkzeug eingeschlossenen Haltemittel ausgestattet ist. Das Haltemittel dient dazu, den Teilbereich durch Anlegen einer gegebenenfalls auch einstellbaren Anpresskraft vorübergehend zu fixieren. Als Wiederlager dient dabei eine ortsfeste oder gemeinsam mit dem Transportband bewegliche Auflage des beispielsweise als Transportband oder Modulband ausgeführten Fördermittels. Die Zustellbewegung des Stanzwerkzeuges erfolgt vorzugsweise mittels einer Gewindespindel oder eines hydraulischen oder pneumatischen Stellmittels gegenüber einer Ober- oder Unterseite des Materialstreifens und erfolgt dabei vorzugsweise weggesteuert derart, dass unterhalb bzw. oberhalb des Fremdkörpers oder der Fehlstelle liegende Schichten nicht oder nur in geringem Maße von dem Werkzeugeingriff betroffen sind. Sobald das Stanzwerkzeug die vorbestimmte Sollposition als Solltiefe in dem Materialquerschnitt erreicht hat, kann dieses zugleich noch in eine umlaufende oder reversierende Drehbewegung um die Zustellachse versetzt werden, um so die Trennung des Teilbereiches des Materials zu optimieren. Selbstverständlich ist die Vorrichtung nicht auf ein einziges Stanzwerkzeug beschränkt. Vielmehr können verschiedene Stanzwerkzeuge unterschiedlicher Größe zum Einsatz kommen. Ebenso sind im Durchmesser einstellbare Stanzwerkzeuge zur optimalen Anpassung an den Fremdkörper oder die Fehlstelle realisierbar.

Eine weitere, ebenfalls besonders vorteilhafte Abwandlung der Erfindung wird auch dadurch erreicht, dass die Ausscheidevorrichtung einen in dem zu entfernenden Teilbereich innerhalb des Stanzwerkzeuges angeordneten, gegen eine Oberfläche des Materials anlegbaren Gegenhalter mit einer Anlagefläche aufweist, durch die der von dem Material getrennten Teilbereich fixierbar ist und die zugleich eine zum Transportieren und Entfernen des Teilbereiches geeignete Beschaffenheit aufweist. Die Anlagefläche kann hierzu in die Oberfläche des Teilbereiches einbringbare Vorsprünge aufweisen, durch die ein lösbares mechanisches Verklammern durch eine kraft- oder formschlüssige Verbindung oder durch Adhäsion erreicht wird. Somit kann der Teilbereich problemlos zu einer Entsorgungsstation transportiert werden.

Besonders praxisnah ist es zudem auch, wenn die Ausscheidevorrichtung gemeinsam mit dem Transportmittel insbesondere translatorisch in Richtung der Haupterstreckungsebene des Materials in zueinander senkrechten Achsen beweglich ist. Hierzu eignet sich beispielsweise eine Ausscheidevorrichtung in Portalbauweise, die den Materialstreifen bzw. das Fördermittel überspannt. Alternativ kann die Ausscheidevorrichtung auch mit einem Schwenkarm für das Stanzwerkzeug ausgestattet sein.

Eine besonders bevorzugte Abwandlung der Erfindung wird dadurch erreicht, dass die Vorrichtung eine Einrichtung zur Messung der Dichte des Materials aufweist, um so das Stanzwerkzeug in optimaler Weise in Abhängigkeit der erfassten Messwerte steuern zu können. Darüber hinaus dient die Dichtemessung auch der Erfassung von Fehlstellen des Materials aufgrund einer von einem Sollwert abweichenden Dichte mit im Übrigen unveränderten Materialparametern. Diese können anderenfalls in einem späteren Erzeugnis zu inhomogenen Eigenschaften führen und werden daher ebenso wie Fremdkörper mittels der erfindungsgemäßen Ausscheidevorrichtung entfernt.

Weiterhin ist es von Vorteil, wenn die Vorrichtung eine Einrichtung zur Messung der Materialstärke in der Querschnittsebene aufweist, sodass der zu entfernende Teilbereich so berechnet werden kann, dass eine Durchbrechung des Materials weitgehend vermieden werden kann.

Vorzugsweise werden vor der Entfernung des Fremdkörpers die Koordinaten des Fremdkörpers oder der Fehlstelle in der Haupterstreckungsebene und/oder der Querschnittsebene des Materials erfasst. Hierdurch wird es möglich, den zu entfernenden Teilbereich auf ein notwendiges Minimum zu beschränken, indem sowohl die Tiefe ausgehend von einer oberen oder unteren Oberfläche des Materials sowie der Randabstand von zumindest einem seitlichen Rand erfasst werden.

Die erfindungsgemäße Aufgabe wird weiterhin noch mit einem Verfahren zur radioskopischen Untersuchung eines insbesondere kontinuierlich mittels eines Fördermittels zugeführten Materials mit einem wesentlichen Bestandteil aus Kautschuk oder Kunststoff dadurch gelöst, dass die gesamte Querschnittsfläche des Materials durchleuchtet wird und somit in dem Material vorhandene Fremdkörper oder Fehlstellen erfasst und mittels einer Ausscheidevorrichtung zusammen mit einem den Fremdkörper oder Fehlstellen einschließenden Teilbereich der Querschnittsfläche von dem Restmaterial getrennt werden. Hierdurch wird es erstmals möglich, Fremdkörper oder Fehlstellen gezielt aus dem Material zu entfernen, ohne dass hierzu der Materialstrang unterbrochen werden muss. Vielmehr wird in dem Teilbereich lediglich ein Volumen des Materials entfernt, welches wenig größer bemessen ist als der Fremdkörper. Dabei kann sogar die Kontur des Fremdkörpers oder der Fehlstelle berücksichtigt und die Schnittführung beim Herauslösen des Fremdkörpers oder der Fehlstelle entsprechend abgestimmt werden. Anschließend wird das Material, vorzugsweise unterbrechungsfrei, einer Einspeiseöffnung eines Extruders zugeführt.

Die erfindungsgemäße Aufgabe wird weiterhin noch mit einem Verfahren zur radioskopischen Untersuchung eines Materials dadurch gelöst, dass die Dichte mittels eines Doppelröntgenabsorptiometrie- oder Dual-Energy-Verfahrens ermittelt wird und die so gewonnenen Messwerte zur Steuerung oder Regelung eines nachfolgenden Extrusionsprozesses, dem das Material zugeführt wird, verwendet werden. Durch den Einsatz eines an sich bekannten Doppelröntgenabsorptiometrie-Verfahrens lässt sich die Zusammensetzung des Materials und insbesondere seine Dichte zuverlässig bestimmen. Hierzu werden zwei Aufnahmen mit unterschiedlicher Röntgenenergie gemacht. Die vergleichende Betrachtung der beiden Messungen erlaubt dabei die Bestimmung der Dichte in beliebigen Materialbereichen oder des gesamten Materials.

Die Dichte kann auch über eine einfache radioskopische Untersuchung und eine Dickenmessung entlang der Querschnittsebene bestimmt werden. Auch die Querschnittsermittlung kann zur Steuerung oder Regelung eines nachfolgenden Extrusionsprozesses verwendet werden. Der ermittelte Querschnitt und die Vorschubgeschwindigkeit des Materialstreifens können auf Basis der Extrusionsparameter (Drehzahl, Temperatur, Druck, Stiftkonfiguration, Materialeigenschaften, Schneckengeometrie, u. a.) darüber hinaus zur Erkennung des Verschleißes des Extruders herangezogen werden.

Die Erfindung lässt verschiedene Ausführungsformen zu. Zur weiteren Verdeutlichung ihres Grundprinzips ist eine davon in der Zeichnung dargestellt und wird nachfolgend beschrieben. Diese zeigt in
- Fig. 1: eine Draufsicht auf eine erfindungsgemäße Vorrichtung mit einer Ausscheidevorrichtung;
- Fig. 2: die Ausscheidevorrichtung in einer Seitenansicht.

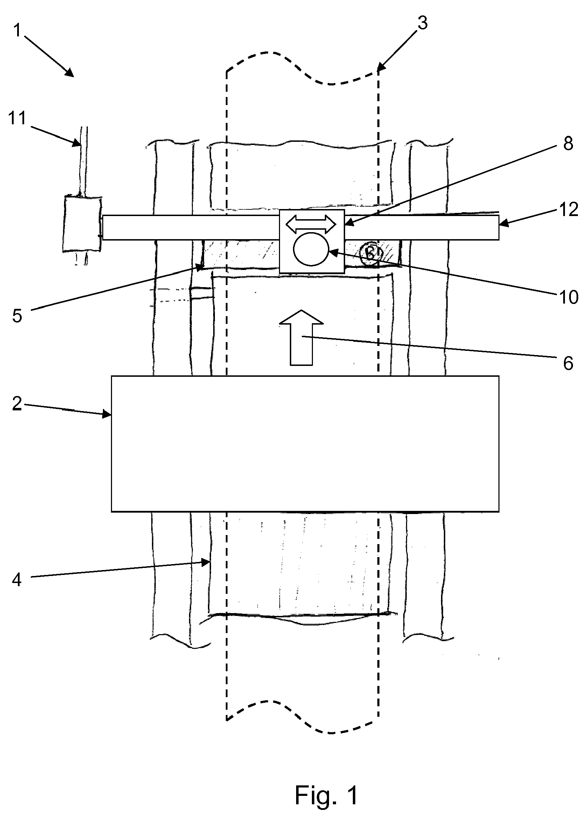

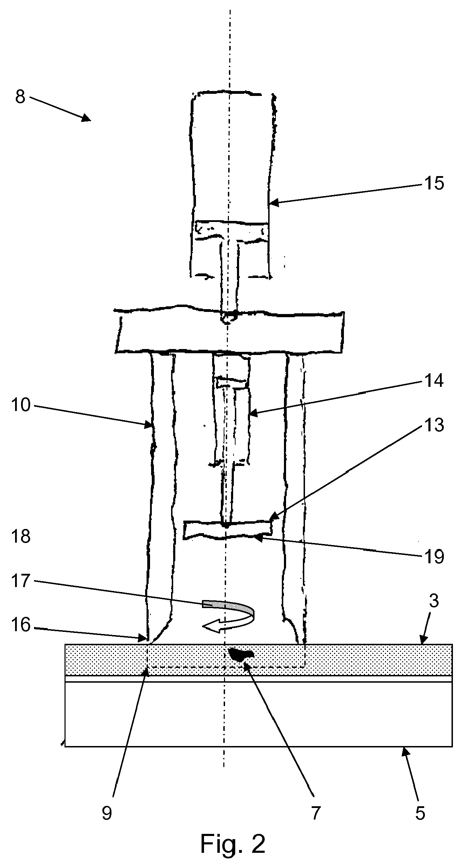

Die erfindungsgemäße Vorrichtung 1 mit einer radioskopischen Messeinrichtung 2 wird nachstehend anhand der Figuren 1 und 2 näher erläutert. Die Vorrichtung 1 dient der Untersuchung eines kontinuierlich durchlaufenden streifenförmigen Materials 3 aus Kautschuk. Der Zuführung dient ein als Transportband ausgeführtes Fördermittel 4, auf dessen umlaufend antreibbaren Auflage 5 das Material 3 in Pfeilrichtung 6 transportiert und zur Weiterverarbeitung einer nicht weiter dargestellten Einspeiseöffnung eines Extruders zugeführt wird. Während der Bewegung wird das streifenförmige Material 3 von der radioskopischen Messeinrichtung 2 durchleuchtet und dabei die gesamte Querschnittsfläche erfasst, sodass in dem Material 3 vorhandene Fremdkörper 7 oder Fehlstellen nach ihrer Position und Orientierung erfasst werden. Zur Erfassung des gesamten Querschnittes ist dabei lediglich eine einzige Bestrahlung ausreichend. Weiterhin ist die Vorrichtung 1 mit einer Ausscheidevorrichtung 8 zur Entfernung des zuvor identifizierten Fremdkörpers 7 oder der Fehlstelle anhand der ermittelten Koordinaten zusammen mit einem den Fremdkörper 7 oder die Fehlstelle einschließenden Teilbereich 9 bezogen auf die Haupterstreckungsebene sowie der Querschnittsfläche des Materials 3 ausgestattet. Die Ausscheidevorrichtung 8 ermöglicht das Entfernen dieses Teilbereiches 9 während der Vorschubbewegung des Materials 3, indem das als Stanzwerkzeug ausgeführte Werkzeug 10 der Ausscheidevorrichtung 8 mittels entsprechender Führungen 11, 12 in Vorschubrichtung sowie in Querrichtung hierzu entsprechend synchron bewegt wird.

Die in Figur 2 in einer Seitenansicht gezeigte Ausscheidevorrichtung 8 mit dem hohlzylindrischen Werkzeug 10 schließt einen gegenüber dem Material 3 vorspannbaren Gegenhalter 13 umfangsseitig ein. Zum Entfernen des Teilbereiches 9 wird zunächst der Gegenhalter 13 mittels eines Hubkolbens 14 auf das Material 3 abgesenkt und dieses dadurch gegen die Auflage 5 des mitlaufenden Fördermittels 4 angepresst. Anschließend wird das Werkzeug 10 mittels eines weiteren Hubkolbens 15 weggesteuert abgesenkt bis eine Schneidkante 16 die Materialebene des eingeschlossenen Fremdkörpers 7 erreicht hat. Der Zustellung überlagert oder im Anschluss daran erfolgt eine Drehbewegung 17 des Gegenhalters 13 sowie des Werkzeuges 10 um die Zustellachse 18, durch die sodann der Teilbereich 9 von dem verbleibenden Material 3 getrennt wird. Um den abgetrennten Teilbereich 9 entsorgen zu können, ist der Gegenhalter 13 außenseitig mit einer eine Strukturierung aufweisenden Anlagefläche 19 ausgestattet, an welcher der ausgestanzte Teilbereich 9 vorübergehend haftet und sich dadurch leicht einer Entsorgung zuführen lässt.

**BEZUGSZEICHENLISTE**

| | | | |
|---|---|---|---|
| 1 | Vorrichtung | 16 | Schneidkante |
| 2 | Messeinrichtung | 17 | Drehbewegung |
| 3 | Material | 18 | Zustellachse |
| 4 | Fördermittel | 19 | Anlagefläche |
| 5 | Auflage | | |
| | | | |
| 6 | Pfeilrichtung | | |
| 7 | Fremdkörper | | |
| 8 | Ausscheidevorrichtung | | |
| 9 | Teilbereich | | |
| 10 | Werkzeug | | |
| | | | |
| 11 | Führung | | |
| 12 | Führung | | |
| 13 | Gegenhalter | | |
| 14 | Hubkolben | | |
| 15 | Hubkolben | | |

## Patentansprüche

1. Vorrichtung (1) mit einer radioskopischen Messeinrichtung (2) zur Untersuchung eines insbesondere kontinuierlich durchlaufenden, streifenförmigen Materials (3) mit einem wesentlichen Bestandteil aus Kautschuk oder Kunststoff, wobei das Material (3) mittels eines Fördermittels (4) zuführbar ist, **dadurch gekennzeichnet, dass** die Messeinrichtung (2) zur Erfassung der gesamten Querschnittsfläche ausgeführt ist, sodass in dem Material (3) vorhandene Fremdkörper (7) oder Fehlstellen nach ihrer Größe, Position und/oder Orientierung in dem Material (3) erfassbar sind und dass die Vorrichtung (1) mit einer Ausscheidevorrichtung (8) zur Entfernung des Fremdkörpers (7) oder der Fehlstelle aufgrund der erfassten Daten zusammen mit einem den Fremdkörper (7) oder die Fehlstelle einschließenden Teilbereich (9) der Querschnittsfläche ausgestattet ist.

2. Vorrichtung (1) nach Anspruch 1, **dadurch gekennzeichnet, dass** die Ausscheidevorrichtung (8) zur Entfernung des Teilbereiches (9) während einer Vorschubbewegung des Materials (3) ausgeführt ist.

3. Vorrichtung (1) nach den Ansprüchen 1 oder 2, **dadurch gekennzeichnet, dass** die Ausscheidevorrichtung (8) zumindest ein Werkzeug (10), insbesondere zumindest ein Stanzwerkzeug zum Trennen des Teilbereiches (9) aufweist.

4. Vorrichtung (1) nach zumindest einem der vorhergehenden Ansprüche, **dadurch gekennzeichnet, dass** die Ausscheidevorrichtung (8) einen in dem zu entfernenden Teilbereich (9) angeordneten, gegen eine Oberfläche des Materials (3) anlegbaren Gegenhalter (13) mit einer Anlagefläche (19) aufweist, durch die der Teilbereich (9) vor und/oder nach dem Trennen fixierbar ist.

5. Vorrichtung (1) nach zumindest einem der vorhergehenden Ansprüche, **dadurch gekennzeichnet, dass** die Ausscheidevorrichtung (8) synchron zu dem Material (3) insbesondere translatorisch beweglich ist.

6. Vorrichtung (1) nach zumindest einem der vorhergehenden Ansprüche, **dadurch gekennzeichnet, dass** die Vorrichtung (1) eine Einrichtung zur Messung der Dichte des Materials (3) aufweist.

7. Vorrichtung (1) nach zumindest einem der vorhergehenden Ansprüche, **dadurch gekennzeichnet, dass** die Vorrichtung (1) eine Einrichtung zur Messung der Materialstärke in der Querschnittsebene des Materials (3) aufweist.

8. Vorrichtung (1) nach zumindest einem der vorhergehenden Ansprüche, **dadurch gekennzeichnet, dass** die Koordinaten des Fremdkörpers (7) oder der Fehlstelle in der Haupterstreckungsebene und/oder der Querschnittsebene des Materials (3) erfassbar sind.

9. Verfahren zur radioskopischen Untersuchung eines insbesondere kontinuierlich zugeführten Materials (3) mit einem wesentlichen Bestandteil aus Kautschuk oder Kunststoff, **dadurch gekennzeichnet, dass** die gesamte Querschnittsfläche des Materials (3) durchleuchtet wird und dadurch in dem Material (3) vorhandene Fremdkörper (7) oder Fehlstellen erfasst und mittels einer Ausscheidevorrichtung (8) zusammen mit einem den Fremdkörper (7) oder die Fehlstelle einschließenden Teilbereich (9) der Querschnittsfläche von dem verbleibenden Material (3) getrennt werden.

10. Verfahren zur radioskopischen Untersuchung eines insbesondere kontinuierlich mittels eines Fördermittels (4) zugeführten Materials (3) mit einem wesentlichen Bestandteil aus Kautschuk oder Kunststoff, **dadurch gekennzeichnet, dass** die Dichte mittels eines Doppelröntgenabsorptiometrie- oder Dual-Energy-Verfahrens ermittelt wird und die so gewonnen Messwerte zur Steuerung oder Regelung eines nachfolgenden Extrusionsprozesses verwendet werden.
